# EUROPEAN PATENT APPLICATION

(11) **EP 2 921 844 A1**
(43) Date of publication of application: **23.09.2015**
(21) Application number: 13855687.3
(22) Date of filing: 07.11.2013
(51) Int. Cl.: G01N 21/64, G01N 1/44

(54) **METHOD AND INSTRUMENT FOR SIMULTANEOUSLY MEASURING MERCURY AND CADMIUM BY DIRECT SAMPLE INJECTION**

(30) Priority: 13.11.2012 CN 201210451541
(71) Applicant: Beijing Jitian Instrument Co., Ltd., Beijing 100015 (CN)
(72) Inventor: LIU, Jixin, Beijing 100015 (CN); FENG, Li, Beijing 100015 (CN); ZHENGN, Fengxi, Beijing 100015 (CN); LU, Dong, Beijing 100015 (CN); LI, Junwei, Beijing 100015 (CN)
(74) Representative: Schwabe - Sandmair - Marx
(86) International application number: PCT/CN2013/001351
(87) International publication number: WO 2014/075385

(57) **Abstract**

A method and instrument for simultaneously measuring mercury and cadmium by direct sample injection without the need of sample digestion comprising the steps of: heating a sample in an oxygen atmosphere, with the temperature being controlled as 120-500°C, so that most of the Hg and decomposition products in the sample are evolved; carrying Hg and evolved substances by an air flow into a tubular catalyst furnace for further decomposition, and amalgamating and absorbing Hg with gold while Cd still exists in the sample; placing the treated sample in a pyrolysis furnace for further thermal decomposition, so that Cd and residual Hg in the sample are evaporated at a high temperature; carrying same by argon gas into a cadmium atom collection trap, selectively trapping cadmium using a tungsten wire, and absorbing the residual mercury using a mercury collection trap; and in an argon-hydrogen atmosphere, heating the tungsten wire or a molybdenum wire and gold amalgam in turn to release Cd and Hg, and carrying same by argon-hydrogen gas into an atomic fluorescence spectrometer for on-line detection. On the basis of realizing the function of simultaneous measurement of Hg and Cd in a sample, the entire structure of a detection instrument is simplified, realizing the minimization, facilitating conventional analysis and urgent demands, being capable of being loaded on a vehicle for field application, and having a prospect of application in on-site detection.

## Description

### Technical field

The present invention relates to the field of heavy metal measurement technologies, and in particular, to a method and instrument for simultaneously measuring mercury and cadmium by direct sample injection without the need of sample digestion.

### Background

Along with the developments of the industry, agriculture and mining industry, more and more heavy metals contaminate the living environment, living articles, foods and medicines, which severely pollute the environment and are severely hazardous to human health. Pollutions made by mercury (Hg) and cadmium (Cd) are especially paid attention to since they have carcinogenic, teratogenic and mutational effects when being accumulated in human or animal bodies, and corresponding detection technologies are an important field being developed by researchers competitively.

Currently, the detection technologies include analysis technologies such as ultraviolet-visible spectrophotometry, atomic absorption spectroscopy, atomic fluorescence spectrometry, plasma emission spectrometry, inductively coupled plasma emission spectrometry, plasma mass spectrometry, and high-performance liquid chromatography. In the above methods, the ultraviolet-visible spectrophotometry is not suitable for simultaneous measurement of multiple elements; the atomic absorption spectroscopy has a narrow linear scope and can measure merely one element a time; the atomic fluorescence spectrometry has a simple spectrum with less overlapped spectral lines, and is an atomic spectrum technology having a rapid development; the inductively coupled plasma-atomic emission spectrometry has a severe spectral interference, and is suitable for measuring different elements with a large content difference; and the high-performance liquid chromatography, when being used for measuring a heavy metal, uses precolumn derivatization to enable the heavy metal element and an organic reagent to generate a stable colored complex, separates the colored complex by using a chromatographic column, and performs measurement by using an ultraviolet-visible photometric detector, so that the steps are complicated, and the technology can merely detect a single item and is time consuming for the detection. In addition to the different adaptation factors, many methods are difficult to be promoted and popularized massively in detection fields of foods, medicines and the like due to factors such as low sensitivity, poor selectivity, low recovery rate, complicated operation process or expensive instrument used, and high requirements on the quality of an operator, and are difficult to be widely applied to detection in a short time.

In addition, most detection methods are limited to dissolving a sample into a liquid before measurement on a machine, and the two elements Hg and Cd are generally not easily to be extracted by using the same method, the time used for analyzing the two elements is very long, the process is very complicated, and matrix interference is always a big problem for directly performing atomic spectrum detection analysis technology. After matrix correction, the influence may be alleviated; however, this problem cannot be solved fundamentally. In recent years, for detection analysis of Hg, there are methods and commercial instruments for direct sample injection analysis of liquid, solid powder or gas available in the market, the complicated pre-processing process is omitted, which greatly reduces the analysis time. Likewise, there are also related documents, records and commercial instruments for direct sample injection and analysis of Cd. The importance and necessity of rapid measurement of two elements Hg and Cd in the field of analysis and detection can thus be seen. The direct sample injection analysis of Hg and the direct sample injection analysis of Cd are different analysis measures, and respective measurements of Hg and Cd are implemented by using two kinds of totally different principles, methods and instruments. The commercial instruments for separately analyzing the two elements Hg and Cd are instrument apparatuses using atomic absorption spectrometry (AAS) and atomic fluorescence spectrometry (AFS) as final detection measures, both of them have the characteristic of high sensitivity; however, they cannot implement simultaneous analysis of the two elements Hg and Cd. The key is that in the prior art, there is no report on a method for simultaneous sample injection of two elements Hg and Cd and an instrument for performing simultaneous detection analysis on the two elements.

Therefore, it is a development objective of the detection field and also an outstanding contribution to the prior art to develop an effective detection analysis method that is rapid, accurate and easy to operate, and can satisfy using of broader analysis and detection units.

### Summary

The present invention is directed to overcome the defects of the prior art, fill the blank in the prior art related to a method and instrument of measuring heavy metals Hg and Cd simultaneously, and develop an effective detection analysis method and instrument that are rapid, accurate and easy to operate, and can satisfy using of broader analysis and detection units. On the other hand, on the basis of realizing a perfect function of simultaneous measurement of two elements Hg and Cd in a sample, the structure of the instrument is simplified, realizing the instrument minimization, being capable of being loaded on a vehicle for field application, and facilitating conventional analysis and urgent demands, and having a prospect of application in on-site detection.

A method for simultaneously measuring mercury and cadmium by direct sample injection provided in the present invention includes the following steps:
a sample ashing process: heating a sample in an oxygen atmosphere, with the temperature being controlled at 120-500°C, so that most of the mercury and decomposition products in the sample are evolved; carrying mercury and evolved substances by an air flow into a tubular catalyst furnace for further decomposition, and absorbing the mercury using a mercury collection trap while cadmium still exists in the sample;
pyrolysis and vaporization: placing the treated sample in a pyrolysis furnace for further thermal decomposition, the pyrolysis furnace having the temperature of 1600-2000°C, so that cadmium and residual mercury in the sample are evaporated at a high temperature;
trapping and absorption: carrying same by argon gas into a cadmium atom collection trap and a mercury atom collection trap, selectively trapping cadmium using a tungsten wire or molybdenum wire in the cadmium atom collection trap, and absorbing the residual mercury using the mercury collection trap;
being released and detection analysis: in an argon-hydrogen atmosphere, heating the tungsten wire or molybdenum wire and gold amalgam in turn to release cadmium and mercury, and carrying same by argon-hydrogen gas into an atomic fluorescence spectrometer for detection analysis.

In the method for simultaneously measuring mercury and cadmium by direct sample injection according to the present invention, the pyrolysis furnace is placed in the protection of an argon atmosphere.

In the method for simultaneously measuring mercury and cadmium by direct sample injection according to the present invention, the mercury collection trap contains an adsorbent carrying gold, so as to form gold amalgam with mercury.

In the method for simultaneously measuring mercury and cadmium by direct sample injection according to the present invention, the pyrolysis furnace is a carbon pyrolysis furnace.

In the method for simultaneously measuring mercury and cadmium by direct sample injection according to the present invention, in the hydrogen and argon atmosphere, the hydrogen is 10%-90% (in volume), and the air is provided by an air compressor or a cylinder and is purified.

An instrument for simultaneously measuring mercury and cadmium by direct sample injection according to the present invention is an atomic fluorescence spectrometer, and includes a sample injection system, a light source, an atomizer, a gas path system, an optical path system, a detection system, and a display apparatus. The sample injection system includes a sample injection linkage part, a tubular ashing furnace, a tubular catalyst furnace, an electrothermal evaporation apparatus, a mercury collection trap, and a cadmium atom collection trap. The gas path system is formed by a damping rotor flow meter and a pressure maintaining valve.

In an instrument for simultaneously measuring mercury and cadmium by direct sample injection according to the present invention, the tubular ashing furnace and the tubular catalyst furnace are designed integrally, formed by a heating wire, a thermal insulation component, a power supply, and a temperature control system, and fixed at one side of a bottom plate of the instrument; the electrothermal evaporation apparatus is formed by a shield cover, an evaporation boat, an electrode, an electrode support, and a power supply, where the electrode is located at a lower part of the evaporation boat, disposed on the electrode support and connected to the evaporation boat, the power supply is electrically connected to the electrode, the shield cover and the electrode support form an enclosed space, the evaporation boat is located in the enclosed space, the shield cover and the electrode support are connected movably, an inlet and an outlet are disposed on the shield cover, and the outlet of the shield cover is connected to an inlet of an outer cover of the cadmium atom collection trap through a t-branch pipe; the mercury collection trap is disposed behind the cadmium atom collection trap, sequential release is implemented by using a pinch valve and circuit control, and the power supply supplies the power to heat, so that the captured Hg and Cd are released sequentially and then carried by the argon-hydrogen gas into the atomic fluorescence spectrometer for detection.

In an instrument for simultaneously measuring mercury and cadmium by direct sample injection according to the present invention, the mercury collection trap is formed by a precious metal (such as gold, platinum and rhodium), a support, a filled quartz tube, and a power supply, and the cadmium atom collection trap is formed by a tungsten wire or molybdenum wire, a support, an outer cover, and a power supply, where the outer cover and the support form an enclosed space, the tungsten wire or molybdenum wire is disposed on the support, the tungsten wire or molybdenum wire is located in the enclosed space formed by the outer cover and the support, and an inlet and an outlet are provided on the outer cover.

The instrument for simultaneously measuring mercury and cadmium by direct sample injection according to the present invention further includes an atomic fluorescence spectrometer, which is formed by a tubular ashing furnace, a tubular catalyst combustion furnace, a power supply system, a gas path system, a pyrolysis furnace, a mercury collection trap, a cadmium atom catcher, and a pinch valve.

The instrument for simultaneously measuring mercury and cadmium by direct sample injection according to the present invention is further used in combination with another atomic fluorescence spectrometer, which is another on-line detection instrument independent to the foregoing instrument, and is formed by an atomizer, a detection system, and an optical path system.

The method and instrument for simultaneously measuring mercury and cadmium by direct sample injection according to the present invention have the following beneficial effects: on the basis of realizing a perfect function of simultaneous measurement of two elements Hg and Cd in a sample, the detection method is rapid and accurate, has high sensitivity and easy to operate, and is an effective detection analysis method meeting requirements of broader analysis detection use; the structure of the provided instrument is greatly simplified, so that minimization is easily implemented to be loaded on a vehicle for field application, facilitating conventional analysis and urgent demands, and having a prospect of application in on-site detection.

### Description of the Drawings

FIG. 1 is a schematic connection diagram of an instrument for simultaneously measuring mercury and cadmium by direct sample injection according to the present invention; and
FIG. 2 is a schematic structural diagram of an instrument for simultaneously measuring mercury and cadmium by direct sample injection according to the present invention.

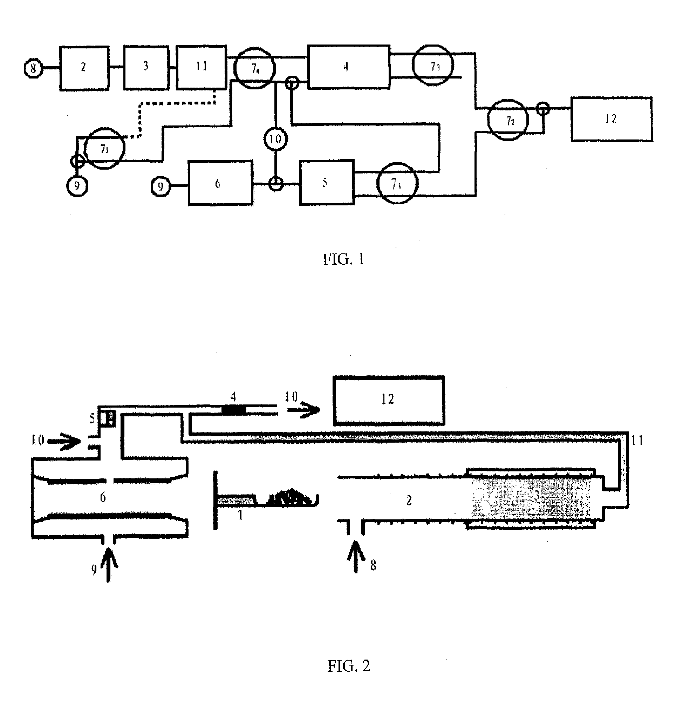

In FIG. 1, 2 indicates a tubular ashing furnace, 3 indicates a tubular catalyst furnace, 4 indicates a mercury collection trap, 5 indicates a cadmium atom collection trap, 6 indicates a pyrolysis furnace, 7 indicates a pinch valve (7₁, 7₂, 7₃, 7₄ and 7₅ respectively indicate pinch valves at different positions), 8 indicates the air, 9 indicates an argon gas, 10 indicates an argon-hydrogen gas, 11 indicates a water collection trap, and 12 indicates an atomic fluorescence spectrometer; and
in FIG. 2, 1 indicates a sample injection linkage component, 2 indicates a tubular ashing furnace, 3 indicates a tubular catalyst furnace, 4 indicates a mercury collection trap, 5 indicates a cadmium atom collection trap, 6 indicates a pyrolysis furnace, 9 indicates an argon gas, 10 indicates an argon-hydrogen gas, 11 indicates a water collection trap, and 12 indicates an atomic fluorescence spectrometer.

### Detailed description

Embodiments of the present invention are explained in detail with reference to FIG. 1 and FIG. 2.

An instrument for simultaneously measuring mercury and cadmium by direct sample injection according to the present invention is formed by a sample injection system, a gas path system, a light source, an atomizer, an optical path system, a detection system, and a display apparatus. The display apparatus is the same as the conventional atomic fluorescence spectrometer. Mutual connection and position relationships between the light source, the optical path system, the detection system, the display apparatus and the atomizer are the same as the conventional atomic fluorescence spectrometer 12. The gas path system and the sample injection system have essentially different structures. The gas path system is formed by a damping rotor flow meter and a pressure maintaining valve. The sample injection system is formed by a tubular ashing furnace 2, a tubular catalyst furnace 3, a water collection trap 11, a pyrolysis furnace 6, a mercury collection trap 4, a cadmium atom collection trap 5, and a series of pinch valves.

An operation method and process of specific detection are described as follows:
A sample is placed in a carbon sample boat of a sample injection linkage component 1 of the sample injection system of the present invention, the sample boat carrying the sample enters the tubular ashing furnace 2 along with the sample injection linkage component 1 to perform an ashing process, in this case, the pinch valve 7₁ is opened, the ashing temperature is accurately controlled by a temperature control system at 50-500°Cto implement program temperature control, thereby meeting ashing conditions of different samples. Organic decomposition released in the ashing process carries most Hg in the sample, and the organic decomposition is carried by the air 8, used as a carrier gas, into the tubular catalyst furnace 3 for further combustion decomposition. The sample is vaporized by the high temperature, the vaporized sample passes through a middle connection apparatus, that is, a water collection trap 11, between the tubular catalyst furnace 3 and the mercury collection trap 1, so as to remove excessive water, and when the decomposition passes through the mercury collection trap 4 at the room temperature or 120°C, the mercury is adsorbed selectively to form gold amalgam. After that, when the temperature of the tubular ashing furnace 2 is reduced to 100-120°C, the sample injection linkage component 1 exits from the tubular ashing furnace 2 and enters the pyrolysis furnace 6; in this case, the pinch valve 7₁ is turned off, the pinch valve 7₄ is turned on, the pyrolysis furnace 6 starts to be heated to 1600-2000°C, cadmium and residual mercury in the sample are evaporated out, and then carried by the argon gas 9 into the cadmium atom collection trap 5 and the mercury collection trap 4. When the pinch valve 7₄ is turned off, the pinch valves 7₁ and 7₂ are turned on, the cadmium atom collection trap 5 is heated to 1600-2000°C, and cadmium atoms are carried by the argon-hydrogen gas 10 to the atomizer; in this case, the atomic fluorescence spectrometer 12 performs online data collection, so as to obtain a signal peak value of Cd. The pinch valve 7₂ is turned off, the pinch valve 7₁ is turned on, and in this case, a gold amalgamation absorbing collection trap is heated to 500-600°C, then, the pinch valves 7₃, 7₄ and 7₅ are turned on simultaneously, the argon-hydrogen gas 10 is used as a carrier gas to carry the mercury to the atomizer of the atomic fluorescence spectrometer 12; in this case, the atomic fluorescence spectrometer collects data to obtain a signal peak value of Hg.

### Embodiment 1

By using 10 mg cabbage (National Standard Material GBW10014) as an example, it is detected, by using the atomic fluorescence spectrometer for simultaneously measuring Hg and Cd by direct sample injection provided in the present invention, that Cd in the sample is 41 mcg/kg, which is within a Cd standard value 35±6 mcg/kg of the standard material; a standard value of Hg is 11.5 mcg/kg, which is in an Hg standard value 10.9±1.6 mcg/kg of the standard material, and the adding standard recoveries of Hg and Cd in high, middle and low levels are all between 90% and 110%.

### Embodiment 2

By using 10 mg rice noodle (National Standard Material GBW10010) as an example, it is detected, by using the atomic fluorescence spectrometer for simultaneously measuring Hg and Cd by direct sample injection provided in the present invention, that Cd in the sample is 85 mcg/kg, which is within a Cd standard value 87±5 mcg/kg of the standard material; a measured value of Hg is 4.8 mcg/kg, which is in an Hg standard value 5.3±0.5 mcg/kg of the standard material, and the adding standard recoveries of Hg and Cd in high, middle and low levels are all between 90% and 105%.

The two specific embodiments clearly show that simultaneously measuring Hg and Cd by direct sample injection by using the method and instrument of the present invention is simple and feasible, and Hg and Cd in a solid sample can be measured accurately.

The above embodiments merely describe preferred implementation manners of the present invention, and are not intended to limit the scope of the present invention. Without departing from the spirit and essential technical solution of the present invention, various replacements or similar technical solutions made by a person of ordinary skill in the art on the present invention shall all fall within the protection scope determined by the claims disclosed in the present invention.

The present invention is not limited to the above embodiments, and in addition to the above embodiments, the present invention may further have other implementation manners, and any technical solution using the conception disclosed in the present invention, equivalent replacement or equivalent variation shall fall within the protection scope of the present invention.

## Claims

1. A method for simultaneously measuring mercury and cadmium by direct sample injection, comprising:
a sample ashing process: heating a sample in an oxygen atmosphere, with the temperature being controlled at 120-500°C, so that most of the mercury and decomposition products in the sample are evolved and evaporated; carrying mercury and evolved substances by an air flow (8) into a tubular catalyst furnace (3) for further decomposition, and absorbing the mercury using a mercury collection trap (4) while cadmium still exists in the sample;
pyrolysis and vaporization: placing the treated sample in a pyrolysis furnace (6) for further thermal decomposition, the pyrolysis furnace (6) having the temperature of 1600-2000°C, so that cadmium and residual mercury in the sample are evaporated at a high temperature;
trapping and absorption: carrying evaporated substances by argon gas (9) into a cadmium atom collection trap (5) and a mercury atom collection trap (4) in turn, selectively trapping cadmium using a tungsten wire or molybdenum wire in the cadmium atom collection trap (5), and absorbing the residual mercury using the mercury collection trap (4); and
being released and detection analysis: in an argon-hydrogen atmosphere, heating the tungsten wire or molybdenum wire and gold amalgam in turn to release cadmium and mercury, and carrying same by argon-hydrogen gas (10) into an atomic fluorescence spectrometer (12) for detection analysis.

2. The method for simultaneously measuring mercury and cadmium by direct sample injection according to claim 1, wherein the gold amalgam is formed in the sample ashing process by mercury being absorbed by the mercury collection trap (4) with an adsorbent carrying gold in the mercury collection trap (4).

3. The method for simultaneously measuring mercury and cadmium by direct sample injection according to claim 1, wherein the pyrolysis furnace (6) is placed in the protection of an argon atmosphere.

4. The method for simultaneously measuring mercury and cadmium by direct sample injection according to claim 1, wherein the pyrolysis furnace (6) is a carbon pyrolysis furnace.

5. The method for simultaneously measuring mercury and cadmium by direct sample injection according to claim 1, wherein in the hydrogen and argon atmosphere, the content of hydrogen is 10%-90% (in volume), and the air flow (8) is provided by an air compressor or a cylinder and is purified.

6. An instrument for simultaneously measuring mercury and cadmium by direct sample injection, being an atomic fluorescence spectrometer, and comprising a sample injection system, a light source, an atomizer, a gas path system, an optical path system, a detection system, and a display apparatus, wherein
the sample injection system comprises a sample injection linkage part (1), a tubular ashing furnace (2), a tubular catalyst furnace (3), an electrothermal evaporation apparatus, a mercury collection trap (4), and a cadmium atom collection trap (5); and
the gas path system is formed by a damping rotor flow meter and a pressure maintaining valve.

7. The instrument for simultaneously measuring mercury and cadmium by direct sample injection according to claim 6, wherein the tubular ashing furnace (2) and the tubular catalyst furnace (3) are designed integrally, formed by a heating wire, a thermal insulation component, a power supply, and a temperature control system, and fixed at one side of a bottom plate of the instrument.

8. The instrument for simultaneously measuring mercury and cadmium by direct sample injection according to claim 6, wherein the electrothermal evaporation apparatus is formed by a shield cover, an evaporation boat, an electrode, an electrode support, and a power supply, wherein
the electrode is located at a lower part of the evaporation boat, disposed on the electrode support and connected to the evaporation boat, the power supply is electrically connected to the electrode, the shield cover and the electrode support form an enclosed space, the evaporation boat is located in the enclosed space, the shield cover and the electrode support are connected movably, an inlet and an outlet are disposed on the shield cover, and the outlet of the shield cover is connected to an inlet of an outer cover of the cadmium atom collection trap (5) through a t-branch pipe.

9. The instrument for simultaneously measuring mercury and cadmium by direct sample injection according to claim 6, wherein the mercury collection trap (4) is disposed behind the cadmium atom collection trap (5), sequential release is implemented by using a pinch valve (7) and circuit control; and the power supply supplies the power to heat, so that the captured mercury and cadmium are released sequentially, and then carried by the argon-hydrogen gas (10) into the atomic fluorescence spectrometer (12) for detection.

10. The instrument for simultaneously measuring mercury and cadmium by direct sample injection according to claim 6, wherein the mercury collection trap (4) is formed by a precious metal, a support, a filled quartz tube, and a power supply.

11. The instrument for simultaneously measuring mercury and cadmium by direct sample injection according to claim 10, wherein the precious metal comprises gold, platinum and rhodium.

12. The instrument for simultaneously measuring mercury and cadmium by direct sample injection according to claim 6, wherein the cadmium atom collection trap (5) is formed by a tungsten wire or molybdenum wire, a support, an outer cover, and a power supply, wherein
the outer cover and the support form an enclosed space;
the tungsten wire or molybdenum wire is disposed on the support, and the tungsten wire or molybdenum wire is located in the enclosed space formed by the outer cover and the support; and
an inlet and an outlet are provided on the outer cover.

13. The instrument for simultaneously measuring mercury and cadmium by direct sample injection according to claim 6, further comprising an atomic fluorescence spectrometer, which is formed by a tubular ashing furnace (2), a tubular catalyst combustion furnace, a power supply system, a gas path system, a pyrolysis furnace (6), a mercury collection trap (4), a cadmium atom catcher (5), and a pinch valve (7).

14. The instrument for simultaneously measuring mercury and cadmium by direct sample injection according to claim 6, wherein the instrument for simultaneously measuring mercury and cadmium by direct sample injection further needs to be used in combination with another atomic fluorescence spectrometer (12) for on-line detection, which is independent to the foregoing instrument and is formed by an atomizer, a detection system, and an optical path system.
